# EUROPÄISCHE PATENTANMELDUNG

(11) **EP 1 166 712 A1**
(43) Veröffentlichungstag der Anmeldung: **02.01.2002**
(21) Anmeldenummer: 01250232.4
(22) Anmeldetag: 22.06.2001
(51) Int. Cl.: A61B 5/022, A61B 5/02

(54) **Vorrichtung zur nichtinvasiven Messung und/oder Uberwachung des Blutdrucks**

(30) Priorität: 22.06.2000 DE 10030439
(71) Anmelder: Stier, Axel B., 21369 Nahrendorf (DE)
(72) Erfinder: Stier, Axel B., 21369 Nahrendorf (DE)
(74) Vertreter: Erich, Dieter

(57) **Zusammenfassung**

Die Erfindung betrifft eine Vorrichtung zur nichtinvasiven Messung und/oder Überwachung des Blutdrucks. Aufgabe ist es, eine Messung bzw. Überwachung auch unter mobilen Umständen und während physischer Belastungen des Probanden zu ermöglichen und dabei insbesondere durch akustische Artefakte auftretende Verfälschungen der Messwerte zu eliminieren. Die Messung erfolgt, unter Verwendung einer ein Blutgefäß wechselweise eingeengten und entlasteten Manschette (4), durch akustische Detektierung sowie akusto-elektrische Wandlung der im Blutgefäß auftretenden Korotkoff-Geräusche. Durch Umgebungsgeräusche verursachte Artefakte werden kompensiert, indem diese gesondert detektiert, akusto-elektrisch gewandelt und die hieraus resultierenden elektrischen Signale dem auf das Vorhandensein der Korotkoff-Geräusche auszuwertenden Signal gegenphasig überlagert werden. Erfindungsgemäß werden außerdem durch die Muskeltätigkeit des Probanden verursachte innere Artefakte und/oder Schwankungen des Drucks in der Manschette kompensiert. Die Kompensation der inneren Artefakte erfolgt analog der der äußeren. Zum Ausgleich der Druckschwankungen ist ein Regelungskreis vorgesehen, in welchen zumindest die zur Einengung des Blutgefäßes pneumatisch befüllbare Kammer (6) der Manschette und ein mit ihr über ein Ventil verbundenes Ausgleichsvolumen einbezogen sind.

## Beschreibung

Die Erfindung betrifft eine Vorrichtung zur nichtinvasiven Messung und/oder Überwachung des Blutdrucks, mittels welcher eine einmalige oder wiederholte Ermittlung des systolischen und des diastolischen Blutdrucks eines Probanden nach der Methode von Riva-Rocci durchgeführt wird. Als wesentlich ist hervorzuheben, dass die erfindungsgemäße Vorrichtung eine exakte Messung bzw. zuverlässige Überwachung des Blutdrucks in einem in jeder Hinsicht mobilen Einsatz zulässt. Dabei soll unter einem solchen mobilen Einsatz nicht nur die Messung oder Überwachung des Blutdrucks im nichtstationären, quasimobilen Bereich verstanden werden. Vielmehr geht die Erfindung weit über die Möglichkeit der Blutdruckmessung an wechselnden Orten hinaus. Sie betrifft nicht nur die Transportabilität des Blutdruckmessgerätes und seinen in diesem Sinne quasimobilen Einsatz, sondern die Messung bzw. Überwachung des Blutdrucks unabhängig vom jeweiligen Aufenthaltsort und Bewegungszustand des Probanden sowie seiner eventuellen körperlichen Betätigung.
Bei der Messung des Blutdrucks unterscheidet man invasive und nichtinvasive Verfahren. Nach den invasiven Verfahren erfolgt die Blutdruckmessung unmittelbar in einem Blutgefäß durch Einführung eines speziellen Katheters in das Blutgefäß. Bereits durch die Notwendigkeit eines Eindringens in ein Blutgefäß und die hierdurch bestehenden Anforderungen an eine infektionsvermeidende Sterilität scheiden diese Verfahren im Grunde für den vollständig mobilen Einsatz aus.
Die nichtinvasiven oder unblutigen Verfahren basieren auf einer Messung, bei der durch das Fließen des Blutes und durch den Pulsschlag verursachte messbare Größen erfasst und zur indirekten Bestimmung des Blutdrucks ausgewertet werden. Am weitesten verbreitet ist dabei das auch bei der manuellen Blutdruckmessung angewandte Verfahren nach Riva-Rocci. Hierbei wird an ein Körperglied des Probanden eine Manschette angelegt und diese Manschette durch Aufpumpen mit einem Überdruck beaufschlagt. Der Druck in der Manschette wird bis zur vorübergehenden Abschnürung eines arteriellen Blutgefäßes erhöht. Danach wird der Druck allmählich wieder verringert und gleichzeitig das eingeengte Blutgefäß auskultiert, also mittels eines Stethoskops oder im Falle einer automatisierten Messung mittels eines akusto-elektrischen Wandlers abgehorcht. Sobald der Druck soweit verringert wurde, dass eine nennenswerte Blutmenge das Blutgefäss infolge der Pumpleistung des Herzens in einer turbulenten Strömung wieder passieren kann, lassen sich in den zum Abhören des Blutgefäßes verwendeten Detektoren charakteristische Töne, die so genannten Korotkoff-Töne wahrnehmen. Der zu diesem Zeitpunkt an der immer noch unter erhöhtem Druck stehenden Manschette feststellbare Druck entspricht dem systolischen Blutdruck. Bei einer weiteren, allmählichen Verringerung des Überdrucks in der Manschette geht die zunächst turbulente Strömung des Blutes schließlich in eine laminare Strömung über und die Korotkoff-Töne verstummen. Der im Augenblick des Ausbleibens bzw. Verstummens der Korotkoff-Töne an der Manschette feststellbare Druck entspricht dem diastolischen Blutdruck.
Bei einer automatisierten Messung mittels dieses Verfahrens tritt das Problem auf, dass die vergleichsweise schwachen Korotkoff-Töne leicht durch Störgeräusche unterschiedlichster Ursache überlagert werden können und es hierdurch zu einer Verfälschung des Messergebnisses kommt. Quellen solcher Störgeräusche können in der Umgebung der Messstelle (beispielsweise Verkehrsgeräusche, Geräusche durch Radio oder Fernsehen etc.) zu suchen sein oder aus Bewegungen des Probanden und der damit verbundenen Tätigkeit von Muskeln oder Gelenken resultieren. Insbesondere die Tätigkeit der Muskeln kann darüber hinaus aufgrund der Ausübung einer Druckkraft auf die aufgepumpte Manschette zu Verfälschungen bei der Messung des jeweils in der Manschette herrschenden Druckes führen.
Eine andere bei der automatisierten Messung häufig verwendete Methode ist die oszillometrische Messung. Das Messprinzip ist ähnlich dem zuvor geschilderten, nur dass an Stelle der durch die Strömung des Blutes verursachten Geräusche die durch den Pulsschlag hervorgerufenen Druckänderungen mittels Drucksensoren erfasst werden. Bei diesen Messverfahren tritt zwar eine Verfälschung der Messung durch akustische Artefakte nicht auf, jedoch ist auch die mittels der Drucksensoren durchgeführte Messung relativ störanfällig.
Zur Vermeidung der zuvor geschilderten Probleme ist bereits eine Vielzahl technischer Lösungen vorgeschlagen worden. Diese beziehen sich bei der akustischen, durch Detektion der Korotkoff-Geräusche erfolgenden Messung auf spezielle Filteranordnungen zum Ausfiltern der Störgrößen oder auf Maßnahmen, mit denen erreicht wird, dass die Messung innerhalb eines vermeintlich störungsfreien Zeitraumes (innerhalb eines so genannten Fensters) erfolgt.
Aus der DE 28 32 939 A1 ist ein Verfahren bekannt geworden, bei dem ein zunächst als Korotkoff-Geräusch angesehenes Signal wieder verworfen wird, sofern innerhalb eines gewissen Zeitraumes, welcher einem Puls von etwa 45 Schlägen pro Minute entspricht, kein weiteres Signal detektiert wird. In einem solchen Fall wird dann das zunächst als Korotkoff-Geräusch bewertete Signal als ein Artefakt angesehen, da anderenfalls in der genannten Zeitspanne ein weiterer Pulsschlag feststellbar sein müsste. Durch diese Maßnahme können aber nur Störgeräusche innerhalb eines Frequenzbereiches unterhalb der Pulsfrequenz eliminiert werden.
In der DE 30 14 219 A1 wird ein Blutdruckmesser beschrieben, bei dem das Korotkoff-Geräusch, welches durch ein Mikrophon empfangen wurde, über eine spezielle Filteranordnung geführt wird. Durch die Filteranordnung wird ein großer Teil gegebenenfalls auftretender Störgeräusche ausgefiltert. Nach einem ähnlichen Prinzip arbeitet ein in der DE 31 43 372 A1 dargestelltes Verfahren. Gemäß der in der letztgenannten Schrift offenbarten Lehre wird mittels einer Filteranordnung das Auftreten der Korotkoff-Geräusche anhand eines für sie typischen Schwingungsverlaufs identifiziert. Auch eine in der DE 34 14 085 A1 offenbarte Lösung identifiziert die Korotkoff-Töne anhand des speziell ihnen eigenen Schwingungsspektrums.
Zur Kompensation bei der oszillometrischen Messung auftretender Störsignale wird durch die DE 44 10 297 A1 eine technische Lösung beschrieben. Gemäß der in der Schrift dargestellten Lösung werden gleichzeitig das Nutzsignal mittels eines Druckwandlers und ein das Nutzsignal nicht enthaltendes, durch gegebenenfalls aufgetretene Artefakte verursachtes, ebenfalls mittels eines Drucksensors gewonnenes Signal erfasst. Das ausschließlich die Störgröße enthaltende Signal wird dem Nutzsignal gegenphasig überlagert. Die zur Realisierung des Verfahrens dienende Manschette besteht aus zwei gegeneinander schwingungs- und druckisolierten Messkammern. Dadurch, dass die Kammern vorzugsweise durch eine Hartschale vollständig voneinander entkoppelt sind, lassen sich mit dieser Lösung zwar gegebenenfalls von außen her einwirkende atmosphärische Druckschwankungen eliminieren, jedoch wird nicht erkennbar, inwieweit durch die Muskeltätigkeit verursachte Artefakte hierdurch kompensiert werden können.

Alle vorgenannten Maßnahmen beseitigen bzw. kompensieren jeweils einige der möglichen Störfaktoren, sind aber im Hinblick auf die Genauigkeit der Messergebnisse in vielen Fällen noch nicht ausreichend. Soweit die dargestellten Lösungen auch für den mobilen Einsatz konzipiert sind, betrifft dies immer nur einen quasimobilen Einsatz, d. h. die Möglichkeit auch unabhängig von stationären Einrichtungen, beispielsweise im Heimbereich oder in einem Krankenfahrzeug, Blutdruckmessungen vorzunehmen. Dabei werden aufgrund der weiterhin durch Artefakte bestehenden Probleme jeweils bei der Messung die äußeren Gegebenheiten so gewählt, dass eventuelle Störfaktoren, wie Umgebungsgeräusche oder durch die Bewegung des Probanden verursachte Geräusche, weitestgehend vermieden werden. Im Allgemeinen nimmt der Proband während der Messung daher eine gewisse Ruhehaltung ein. Die Verfahren sind somit nicht geeignet, eine Blutdruckmessung oder -überwachung unter tatsächlich mobilen Umständen, also auch völlig unabhängig vom Bewegungs- oder Belastungszustand des Probanden, durchzuführen. So ist beispielsweise eine Überwachung des Blutdrucks während der physischen Belastung des Probanden durch eine sportliche Betätigung nach diesen Lösungsansätzen nicht möglich. Die zuvor dargestellten, nach der Methode von Riva-Rocci arbeitenden Lösungen verwenden jeweils nur ein Mikrophon.

Aufgabe der Erfindung ist es daher, eine Vorrichtung zu schaffen, mit welcher die nichtinvasive Messung und/oder Überwachung des Blutdrucks unter Vermeidung der vorgenannten Nachteile möglich ist, und zwar unabhängig davon, ob sich der Proband gerade in einer für eine genaue Messung zuträglichen Ruhehaltung befindet, einer körperlichen Beanspruchung unterliegt oder gegebenenfalls zusätzlich dem Einfluss von Umgebungsgeräuschen ausgesetzt ist.

Die Aufgabe wird durch ein Vorrichtung mit den Merkmalen des Hauptanspruchs gelöst. Vorteilhafte Ausgestaltungen bzw. Weiterbildungen der Erfindung sind durch die jeweiligen Unteransprüche gegeben.
Das erfindungsgemäße Verrichtung bedient sich bei der einmaligen oder wiederholten Messung der Methode von Riva-Rocci, wobei die erhaltenen Werte zumindest im Falle einer wiederholten Messung temporär gespeichert werden. Durch Umgebungsgeräusche verursachte äußere Artefakte werden dadurch kompensiert, dass sie gesondert detektiert, akusto-elektrisch gewandelt und die ihnen entsprechenden elektrischen Signale dem auf das Vorhandensein der Korotkoff-Geräusche auszuwertenden Signal gegenphasig überlagert werden. Die erfindungsgemäße Vorrichtung umfasst zu diesem Zweck zumindest eine an einem Körperglied anzulegende Manschette mit einer pneumatisch befüllbaren, das Körperglied zumindest teilweise umfassenden Kammer zur wechselweisen partiellen Einengung und Entlastung eines Blutgefäßes, eine Pumpe zur pneumatischen Befüllung der Kammer, Mittel zur zeitlich gesteuerten Verringerung eines in der Kammer infolge des Aufpumpens der Manschette entstandenen Überdrucks, mehrere akusto-elektrische Wandler (vorzugsweise als Mikrophone), Einheiten zur Phasenverschiebung bzw. -drehung eines elektrischen Wechselsignals der Wandler, einen Signaladdierer, einen Druckmesser zur Messung des pneumatischen Drucks in der Kammer, Mittel zur Speicherung und Anzeige von Messwerten, Mittel zur Bedienung der Vorrichtung, eine Steuer- und Auswerteeinheit, Mittel zur Spannungsversorgung der vorgenannten Einheiten. In erfindungswesentlicher Weise umfasst die Vorrichtung aber außerdem Mittel zur Eliminierung durch Bewegungen des Probanden hervorgerufener Störfaktoren. Die zur Feststellung der Korotkoff-Geräusche und der Detektion der äußeren Artefakte dienenden akusto-elektrischen Wandler der Vorrichtung bilden zwei, jeweils immer mindestens einen Wandler umfassende Wandlergruppen aus, von denen eine auf der dem Körperglied abgewandeten Seite der dieses umfassenden Kammer und die andere auf der dem Körperglied zugewandten Seite der Kammer angeordnet ist. Dabei werden die elektrischen Signale der nicht am Blutgefäß anliegenden Wandlergruppe nach einer durch die Einheit zur Phasendrehung bewirkten 180°-Drehung ihrer Phase mit dem zuvor hinsichtlich seiner Phase durch die Einheit zur Phasenverschiebung um die zur Phasendrehung benötigte Zeit verschobenen Signal der am Blutgefäß anliegenden Wandlergruppe in dem Signaladdierer zusammengeführt. Mittels der erfindungsgemäßen Vorrichtung werden also gleichzeitig alle akustischen Artefakte, unabhängig von der Quelle ihrer Entstehung oder die äußeren Artefakte und die aufgrund der Körperbewegung verursachten Druckschwankungen in der Messkammer der Manschette oder aber sowohl alle akustischen Artefakte und die Druckschwankungen in der Messkammer kompensiert. Durch welche Kompensationsmöglichkeit die in jedem Falle erfolgende Kompensation der äußeren Artefakte ergänzt wird, hängt von den Erfordernissen des Einzelfalls und der konkreten Ausbildung der zur Erfüllung dieser jeweiligen Erfordernisse ausgelegten Vorrichtung ab.
Bei einer möglichen Ausbildung der Verrichtung ist zur Eliminierung von bei der Bewegung des Probanden auftretenden inneren Artefakten eine mindestens einen elektro-akustischen Wandler umfassende Wandlergruppe auf der dem Körperglied zugewandten Seite der Kammer, jedoch dem bei der Messung wechselweise partiell eingeengten Blutgefäß gegenüberliegend, angeordnet. Die aus der Wandlung der von dieser Wandlergruppe detektierten akustischen Signale resultierenden elektrischen Signale werden analog den Signalen der Wandlergruppe zur Detektion der äußeren Artefakte zunächst einer Einheit zur Phasendrehung zugeführt und nach erfolgter 180°-Phasendrehung gemeinsam mit den phasengedrehten Signalen der äußeren Artefakte sowie mit den phasenverschobenen, eigentlichen Messsignalen auf den Signaladdierer geführt. Durch das Zusammenführen der Signale in dem Signaladdierer werden aufgrund der Phasendrehung der das Nutzsignal nicht enthaltenden und ausschließlich durch Störgrößen verursachten Signale die anderenfalls durch die Artefakte bedingten Signalverfälschungen vermieden. Die Phasenverzögerung des Signals der am Blutgefäß anliegenden Wandlergruppe, also des das eigentliche Nutzsignal enthaltenden Signals, ist erforderlich, um die durch die Phasendrehung der Signale der anderen Wandlergruppen entstehende geringfügige zeitliche Verzögerung auszugleichen. Die äußere auf der dem Körperglied abgewandten Seite der zur Einengung des Blutgefäßes aufpumpbaren Kammer positionierte Wandlergruppe detektiert alle durch Umgebungsgeräusche bedingten akustischen Artefakte. Dadurch, dass eine der auf der Innenseite der Kammer angeordneten Wandlergruppen dem Blutgefäß, an welchem die Messung erfolgt, gegenüberliegt, werden durch diese Wandlergruppe ausschließlich interne akustische Artefakte, wie sie beispielsweise durch die Bewegung des Ellenbogengelenks entstehen, registriert. Durch die Registrierung einerseits des mit den Artefakten behafteten Messsignals und der andererseits ausschließlich Artefakte enthaltenden Signale und deren gegenphasige Überlagerung mit dem Messsignal werden nahezu alle akustischen Störungen unterdrückt.
Eine andere Ausbildungsform der Vorrichtung weist neben den Einheiten zur Kompensation der äußeren Artefakte im radial äußeren Bereich der Manschette ein die Kammer umgebendes und mit dieser über ein Ventil verbundenes Ausgleichsvolumen auf. Dabei bilden die Messkammer, das Ausgleichsvolumen und das Ventil einen Regelungskreis zur Kompensation von durch das Anspannen und Erschlaffen der Muskulatur bei Bewegungen des Probanden in der Kammer auftretenden Druckschwankungen. Die durch die Muskeltätigkeit eintretenden Druckschwankungen in der Manschette werden durch das mit der Messkammer verbundene Ausgleichsvolumen ausgeglichen. Diese Ausgleichskammer kann im Sinne der Erfindung mit der Messkammer durch ein Flatterventil, ein in anderer Weise definiert mechanisch und ohne Hilfsenergie vorgespanntes Ventil oder durch ein steuerbares Ventil verbunden sein. Im letztgenannten Fall erfolgen die Regelung und der Ausgleich eines gegebenenfalls durch die Körperbewegung kurzzeitig entstehenden Überdrucks in der Manschette vermittels eines das regelbare Ventil ansteuernden so genannten PID-Reglers, welcher Teil der Steuer- und Auswerteeinheit ist.
Die wiederholte Messeung des Blutdrucks zu seiner Überwachung kann auf unterschiedliche Weise bewirkt werden. Gemäß einer Möglichkeit verfügt die Vorrichtung zu diesem Zweck über Mittel für eine zeitzyklisch gesteuerte wiederholte automatisierte Ermittlung des systolischen und diastolischen Blutdruck. Bei einer anderen Ausbildungsform der Erfindung sind in der Vorrichtung Mittel für eine hinsichtlich des Wiederholungsintervalls zufallsgesteuerte wiederholte automatisierte Ermittlung des Blutdrucks vorgesehen.
In besonderen Einsatzfällen, beispielsweise bei der gezielten Überwachung des Blutdrucks während körperlicher Belastungsphasen, werden die zur Ermittlung der Blutdruckwerte erforderlichen Detektierungs- und Messvorgänge automatisiert bei Feststellung einer vorgebbaren Erhöhung der Pulsfrequenz gestartet. Noch spezieller ausgestaltet ist diese Variante dadurch, dass der Beginn der Messung im Falle einer Erhöhung der Pulsfrequenz bei der Feststellung einer Verringerung des Anstiegs, also unmittelbar dem Moment des vermeintlich steilsten Anstiegs der Pulsfrequenz folgend, ausgelöst wird. Eine für die beiden letztgenannten Einsatzfälle bzw. Anforderungen ausgebildete Vorrichtung verfügt über Mittel für eine automatisiert ereignisgesteuerte wiederholte Ermittlung des Blutdrucks. Bei diesen Mitteln handelt es sich um einen zusätzlichen Pulsdetektor zur Feststellung der Pulsfrequenz, der über die Steuer- und Auswerteeinheit (und gegebenenfalls - sofern diese Funktion nicht bereits durch die Steuer- und Auswerteeinheit selbst bereitgestellt wird - eine zusätzliche Vergleicherschaltung) mit einem Speicher zur temporären Speicherung von Messwerten gekoppelt ist. In der Steuer- und Auswerteeinheit werden im Falle einer Erhöhung der Pulsfrequenz, der jeweils im Verhältnis zur letzen Pulsfrequenzmessung sich ergebende Anstieg der Pulsfrequenz und ein innerhalb eines vorgebbaren zeitlichen Intervalls gegebener maximaler Anstieg der Pulsfrequenz abgeleitet. Durch die Steuer- und
Auswerteeinheit wird dann bei Feststellung des festegelegten Ereignisses, also beispielsweise des maximalen Anstiegs der Pulsfrequenz bzw. einer Stabilisierung der Pulsfrequenz auf einem erhöhten Niveau der Messvorgang ausgelöst.
Die Vorrichtung ist außerdem vorzugsweise so ausgebildet, dass die Mittel zur Bedienung sowie zur Speicherung und zur Anzeige von Messwerten die Abfrage und Anzeige ausgewählter Messwerte durch den Benutzer bzw. eine Bedienperson zulassen. Dies kann auch die Möglichkeit einer graphischen Darstellung von Messwerten umfassen. Bei einer besonders vorteilhaft ausgestalteten Weiterbildung der Vorrichtung sind weiterhin Möglichkeiten zum Anschluss externer Geräte für das Auslesen und eine weitergehende Verarbeitung von Messwerten vorgesehen. Darüber hinaus können aber auch Anschlüsse für externe Geräte vorgesehen sein, durch welche mittels dafür geeigneter Steuersignale die Messung zur Ermittlung des systolischen oder diastolischen Blutdrucks gestartet oder im Falle einer wiederholten Messung die zeitliche Abfolge der Messungen gesteuert wird. So ist es denkbar, die Vorrichtung mit einem Ergometer oder einem Laufband zu verbinden und die Messung in Abhängigkeit von der physischen Belastung des Probanden bzw. der von ihm an den genannten Geräten erbrachten Leistungen zu starten und/oder zu wiederholen.
Eine sehr komfortable Ausgestaltung der Vorrichtung kann dadurch gegeben sein, dass sie über Mittel verfügt, welche das Anlegen der Manschette in einer zur Wahrnehmung der Korotkoff-Geräusche optimalen Lage unterstützen. Dabei ist es denkbar, durch das Fließen des Blutes verursachte Geräusche zu detektieren und deren Lauter- oder Leiserwerden durch entsprechende Anzeigemittel (ein Zeigerinstrument, unterschiedlich farbige Leuchtdioden o. dgl.) zu signalisieren.
Zur Einengung des Blutgefäßes wird die Manschette vorzugsweise mit einem mit Sicherheit oberhalb des systolischen Blutdrucks liegenden Druck beaufschlagt. Dabei ist es im Sinne der Erfindung vorgesehen, hierfür zunächst einen (Erfahrungs-) Wert vorzugeben und bei wiederholten Messvorgängen anhand der zuvor für den systolischen Blutdruck des Probanden erhaltenen und gespeicherten Werte eine Veränderung dieses maximalen Fülldrucks vorzunehmen. Eine andere Variante besteht darin, das Blutgefäß bereits beim Aufpumpen der Manschette mittels des auch für die spätere Blutdruckmessung verwendeten Detektors auszukultieren und den Druck in der Manschette geringfügig über einen Druck hinaus zu erhöhen, bei dem vom Blut verursachte Strömungsgeräusche nicht mehr wahrnehmbar sind. Bei dieser Variante ist dann vorteilhafterweise bzw. auch aus Sicherheitsgründen ein durch einen maximalen Manschettendruck oder eine maximale Pumpzeit definiertes Abbruchskriterium für das Aufpumpen vorgesehen.
Von Vorteil kann es darüber hinaus sein, wenn die Manschette auch zwischen den Messvorgängen, also innerhalb der Messpausen, mit einem geringen Überdruck beaufschlagt wird. Der Überdruck wird dabei so gewählt, dass er einerseits nicht störend vom Probanden empfunden wird, aber andererseits ein Verrutschen der zum Erhalt möglichst genauer Messergebnisse in exakter Lage angelegten Manschette verhindert. Für ganz spezielle Anwendungsgebiete, beispielsweise auch wiederum im Sport oder in der Luft- und Raumfahrt, kann das Verfahren noch dahingehend weitergebildet sein, dass kurzzeitige, das Messergebnis verfälschende Beschleunigungen des Blutes zu einer vorübergehenden Unterbrechung der Messung führen.

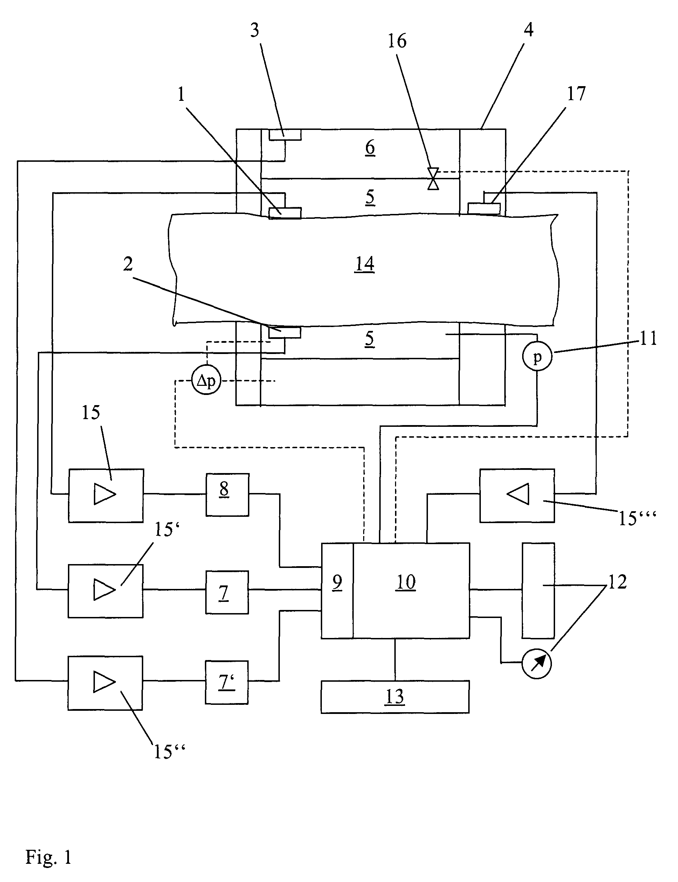

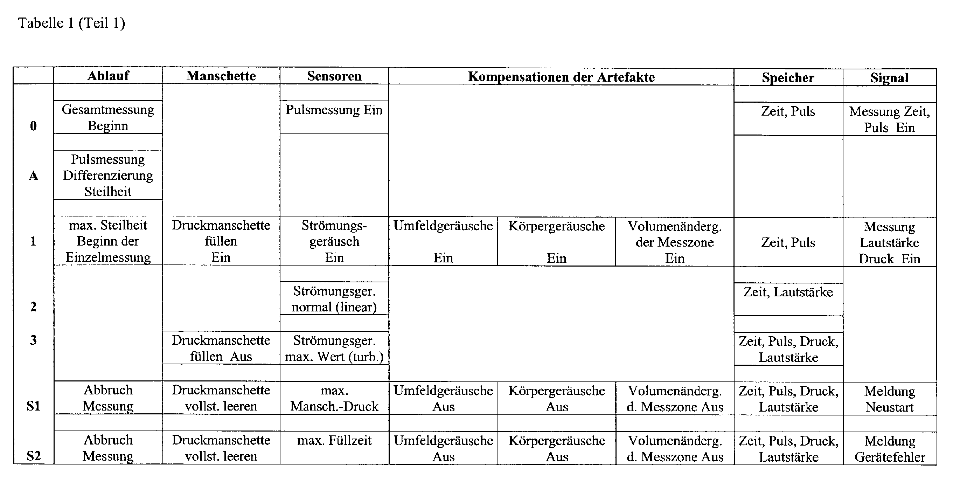

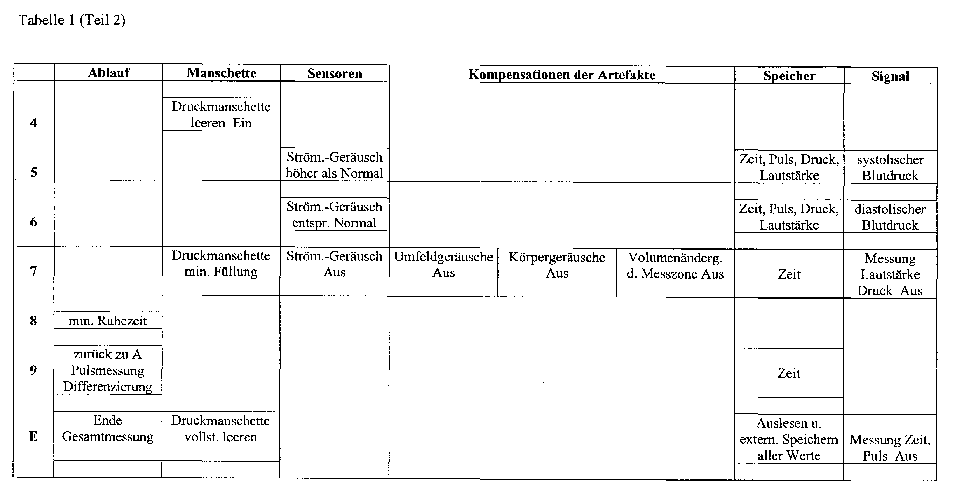

In einem Ausführungsbeispiel der Erfindung soll diese anhand einer Zeichnung (Fig. 1) und einer sich an die nachfolgenden Ausführungen anschließenden Übersicht (Tabelle 1), welche beispielhaft den Verfahrensablauf einer Blutdruckmessung darstellt, nochmals erläutert werden.
In der Fig. 1 ist ein möglicher Aufbau der erfindungsgemäßen Vorrichtung in einer Prinzipdarstellung verdeutlicht, wobei zur besseren Übersicht einiger selbstverständlicher Komponenten wie Pumpe, Stromversorgung (welche vorzugsweise durch Akku's oder Batterien, aber auch je nach Einsatzfall, beispielsweise im stationären Betrieb über ein Netzteil erfolgt) oder Bedienelemente verzichtet wurde.
Wie von anderen Blutdruckmessgeräten her bekannt, weist die Vorrichtung eine Manschette 4 auf, welche zur Messung des Blutdrucks ein Körperglied 14 umfasst und mittels einer Pumpe aufpumpbar ist. In der Regel wird die Messung am Oberarm in der Nähe des Ellenbogens durchgeführt. In erfindungswesentlicher Weise verfügt die in der Figur dargestellte Vorrichtung über drei akusto-elektrische Wandler 1, 2, 3 (beispielsweise Mikrophone). Zwei dieser Wandler 1, 2 befinden sich auf der dem Körperglied 14 zugewandten Seite, der zur wechselweisen Einengung und Entlastung des (nicht dargestellten) arteriellen Blutgefäßes pneumatisch befüllbaren Kammer 5 (Messkammer). Der dritte Detektor bzw. elektro-akustische Wandler 3 befindet sich hingegen im radial äußeren Bereich der Manschette 4, aber jedenfalls auf der dem Körperglied 14 abgewandten Seite der Messkammer 5. Durch diesen Wandler 3 werden äußere akustische Artefakte (Umgebungsgeräusche) erfasst und in ein elektrisches Signal gewandelt. Von den auf der Innenseite der Messkammer positionierten Wandlern 1, 2 dient der Wandler 1 der eigentlichen Blutdruckmessung, also der Erfassung der Korotkoff-Geräusche. Folglich liegt dieser Wandler 1 bei richtig angelegter Manschette 4 an dem für die Messung wechselweise einzuengenden und zu entlastenden Blutgefäß an. Mit ihm werden die durch das Fließen des Blutes verursachten Geräusche erfasst und in ein elektrisches Signal umgewandelt. Allerdings erfasst dieser Wandler 1 darüber hinaus selbstverständlich auch Störgeräusche. Bezogen auf das Körperglied 14, an welchem die Messung erfolgt, liegt dem Wandler 1 ein weiterer Wandler 2 gegenüber, welcher aufgrund seiner Position auf der dem Blutgefäß gegenüberliegenden Seite die vom Blut verursachten Fließgeräusche nicht detektiert, jedoch andere vom Körper des Probanden verursachte Geräusche, welche innere Artefakte darstellen. Diese Geräusche entstehen beispielsweise durch die Muskeltätigkeit bzw. die Bewegung von Gelenken bei einem nicht ruhiggestellten Körperglied 14. Alle von den Wandlern 1, 2, 3 detektierten akustischen Signale werden nach ihrer Umwandlung in elektrische Signale jeweils einem Verstärker 15; 15' 15" 15''' zugeführt. Die Signale der Wandler 2 und 3, welche der Detektion der äußeren bzw. der inneren Artefakte dienen, werden nach ihrer Verstärkung aber zusätzlich über eine Einheit zur Phasendrehung 7, 7' geführt und hierdurch in ihrer Phasenlage um 180° verschoben. Nach der Phasendrehung werden diese Signale dem Signal des der eigentlichen Messung dienenden Wandlers 1 (Messwandler) überlagert. Das von dem Messwandler 1 kommende Signal wurde zuvor zum Ausgleich der für die Phasendrehung der anderen Signale benötigten Zeitspanne in der Einheit 8 geringfügig verzögert. Dadurch, dass die von den akustischen Artefakten erzeugten Signale dem Nutzsignal des Messwandlers 1 gegenphasig überlagert werden, werden diese Signalanteile in dem Nutzsignal kompensiert. Am Ausgang der der Signaladdition dienenden Einheit 9 (nicht gesondert dargestellt, ist vorzugsweise Teil der Steuer- und Auswerteeinheit, welche beispielsweise durch einen Mikrocontroller realisiert wird) steht dann ein Signal zur Verfügung, welches nur noch dem von den Korotkoff-Geräuschen erzeugten Signalverlauf entspricht. Die Manschette 4 der Vorrichtung weist neben den in der Zeichnung nicht dargestellten Mitteln zum Aufpumpen bzw. Reduzieren des Überdrucks (Pumpe, Schläuche, Ventile etc.) zusätzlich noch eine die eigentliche Messkammer 5 umgebende Ausgleichskammer 6 auf. Zwischen der Messkammer 5 und der Ausgleichskammer 6 besteht über ein Ventil 16 eine Verbindung. Beim Aufpumpen und darauffolgenden Ablassen der Luft aus der Messkammer wird die äußere Ausgleichskammer 6 jeweils immer mit einem etwas oberhalb des Drucks in der Messkammer 5 liegendem Druck den dortigen Druckverhältnissen nachgeführt. Praktisch sinnvoll ist dabei ein Überdruck in der Ausgleichskammer bzw. dem Ausgleichsvolumen gegenüber der Messkammer, welcher innerhalb der Messgenauigkeit für den Druck liegt. Durch eine bei der Bewegung des Körpergliedes 14 gegebene Muskeltätigkeit, also das Anspannen der Muskulatur, kann es dazu kommen, dass die Messkammer 5 zusammengedrückt wird und hierdurch in der Messkammer 5 der Druck vorübergehend gegenüber dem nach dem normalen Ablauf vorgegebenen Druck erhöht wird. Verringert sich durch diese Druckerhöhung die zwischen der Messkammer 5 und der Ausgleichs- kammer 6 bestehende Druckdifferenz unter einen vorgegebenen Wert, so wird über das die beiden Kammern 5, 6 verbindende Ventil 16 Luft aus der Messkammer 5 in die Ausgleichskammer 6 gedrückt. Umgekehrt wird bei einem darauffolgenden Erschlaffen des Muskels Luft aus der Ausgleichskammer 6 wieder zurück in die Messkammer 5 gedrückt. Hierdurch wird eine Stabilisierung der Druckverhältnisse in der Messkammer 5 erreicht, ohne dass es dazu erforderlich ist, eventuell mittels der nicht dargestellten Pumpe der Messkammer 5 nochmals Luft zuzuführen. Dieses ist im Hinblick auf den vorgesehenen mobilen Einsatz unter dem Gesichtspunkt der Energieersparnis äußerst vorteilhaft. Die Messkammer 5 und die Ausgleichskammer 6 sowie das sie verbindende Ventil 16 bilden dabei einen Regelungskreis. Je nach Ausgestaltung kann es sich bei dem Ventil 16 um ein regelbares Ventil oder um ein Ventil ohne zusätzliche Hilfsenergie handeln. Im letztgenannten Fall öffnet sich das Ventil bei vorgegebenen Druckdifferenzen aufgrund seiner baulichen Beschaffenheit, beispielsweise dadurch, dass es als ein Flatterventil bzw. ein mechanisch vorgespanntes Ventil ausgebildet ist. Sofern für das Ventil ein regelbares Ventil vorgesehen wird, erfolgt dessen Steuerung durch einen entsprechenden Teil der Steuer- und Auswerteeinheit 10, über die gestrichelt dargestellte Wirkverbindung. Hierbei lässt sich dann ein PID-Regler (Proportional-Iintegral-Differential-Regler) realisieren, welcher eine Stabilisierung des Drucks in der Messkammer in sehr exakter Weise und mit einem günstigen Zeitverhalten ermöglicht. Für den Fall einer sehr starken, durch Körperbewegungen o.ä. verursachten Druckerhöhung in der Messkammer 5 und einer daraus aufgrund des Öffnens des Ventils 16 resultierenden starken Druckerhöhung in der Ausgleichskammer 6, kann zusätzlich die Möglichkeit vorgesehen werden, automatisch Luft aus der Ausgleichskammer 6 in die Umgebung abzublasen.
Sofern die wiederholte Messung des Blutdrucks zu dessen Überwachung über einen längeren Zeitraum ereignisgesteuert, vorzugsweise in Abhängigkeit der Pulsfrequenz erfolgen soll, weist die Vorrichtung zusätzlichen einen Pulsdetektor 17 auf. Dessen Signal wird ebenfalls in ein elektrisches gewandelt und nachfolgend verstärkt. Über die Steuer- und Auswerteeinheit 10 ist zudem eine Verbindung zu Speichereinheiten 13 oder Anzeigeeinheiten 12 hergestellt. Durch die zyklische Erfassung der Pulsfrequenz und die Zwischenspeicherung der dabei gewonnenen Werte ist es dann möglich, den Beginn für eine jeweilige Blutdruckmessung an die Feststellung des vermeintlich stärksten Anstiegs der Pulsfrequenz zu koppeln. Dies ist beispielsweise sinnvoll, wenn das Verhalten des Blutdrucks bei einem Sportler unter Belastung erfasst werden soll. Spezielle Ausgestaltungsformen der Erfindung ermöglichen es zu diesem Zweck auch, erfasste und zwischengespeicherte Messwerte in entsprechenden Anzeigeeinheiten an der Vorrichtung durch Bedienung dafür vorgesehener Bedienelemente sichtbar zu machen, oder gar durch Anschluss der erfindungsgemäßen Vorrichtung an externe Geräte die erhaltenen Messwerte auszulesen und einer weiteren Verarbeitung zuzuführen. Auch eine Kopplung der Vorrichtung an externe Geräte, wie Ergometer oder Laufband, in der Weise, dass durch Signale dieser Geräte die Blutdruckmessung begonnen und/oder der zeitliche Ablauf wiederholter Messungen gesteuert wird, ist möglich.
In dem nachfolgend dargestellten Schema der Tabelle 1 wird der Messablauf, wie er sich für eine ereignisgesteuerte wiederholte Messung des Blutdrucks beispielsweise ergibt, nochmals verdeutlicht. Mit dem Schritt 0 wird die Messung bzw. Überwachung insgesamt gestartet. Dazu wird die Pulsmessung, also der Pulsdetektor 17, eingeschaltet. Die Pulsfrequenz wird zeitlich zyklisch erfasst. Hieraus wird jeweils durch Differenzierung die Steilheit des Anstiegs der Pulsfrequenz ermittelt. Bei Ermittlung der maximalen Steilheit innerhalb eines vorgebbaren Zeitintervalls wird mit einer Blutdruckmessung begonnen. Die Druckmanschette 4 wird mittels der Pumpe gefüllt. Gleichzeitig werden die akustischen Detektoren bzw. akusto-elektrischen Wandler 1, 2, 3 eingeschaltet. Auch der der Druckstabilisierung in der Messkammer 5 dienende Regelungskreis wird, sofern dies beispielsweise im Falle eines mit einem steuerbaren Ventil 16 arbeitenden Regelungskreises erforderlich ist, in Aktion versetzt. Solange normale auf eine laminare Strömung des Blutes in dem Blutgefäß hinweisende Strömungsgeräusche durch den Wandler 1 detektiert werden, wird der Druck in der Manschette 4 weiter erhöht. Beim Übergang der Strömungsgeräusche in ein Spektrum, welches einer turbulenten Strömung entspricht, ist der annähernd maximale zum vollständigen, vorübergehenden Abdrücken des Blutgefäßes erforderliche Druck in der Manschette 4 erreicht. Um sicher oberhalb des systolischen Blutdruckes zu liegen, wird der Druck dann nur noch geringfügig (um ca. 400 bis 600 Pa) weiter erhöht. Danach kann mit dem allmählichen Ablassen der in die Manschette 4 gepumpten Luft die eigentliche Messung erfolgen.
In dem Ablaufschema sind zwischen dem Schritt der maximalen Befüllung der Druckmanschette und des Ablassens der Luft aus der Messkammer 5 noch die Schritte S1 und S2 eingefügt. Diese definieren Abbruchbedingungen während des Befüllens der Manschette 4, um den Probanden vor einem zu hohen Druck zu schützen. Solche Abbruchbedingungen sind zum Einen in einem maximalen Manschettendruck (beispielsweise einem 300 mm Quecksilbersäule entsprechenden Druck) oder einer maximalen Füllzeit definiert. Ist bis zum Erreichen dieser Abbruchkriterien ein Übergang der laminaren Blutströmung in eine turbulente nicht erkannt worden, wird der Messvorgang abgebrochen und die Manschette 4 vollständig bzw. bis auf den das Verrutschen der Manschette 4 verhindernden leichten Überdruck entleert.
Bei einem regulären Ablauf des Messvorgangs wird gemäß Punkt 4 mit dem allmählichen Entleeren der Druckmanschette 4 fortgefahren. Dabei erfolgt entsprechend den Punkten 5 und 6 die Detektion der Korotkoff-Geräusche und die Messung der mit Ihnen korrespondierenden Druckwerte in der Manschette 4. Während dieser ganzen Zeit sind die Detektoren bzw. Wandler 2, 3 zur Erfassung der akustischen Artefakte (äußere und innere akustische Artefakte) eingeschaltet und der Regelungskreis 5, 6, 16 zur Kompensation von Artefakten in Form einer Volumenänderung bzw. Druckererhöhung oder Erniedrigung in der Messkammer 5 aktiviert. In der bereits dargestellten Weise erfolgt mittels der von ihnen gewonnenen Signale und deren phasengedrehte Überlagerung mit dem Nutzsignal eine Kompensation der entsprechenden Störanteile in dem Nutzsignal. Nach der Messung wiederholt sich der Ablauf, beginnend mit Schritt A, unter Einhaltung einer gemäß Schritt 8 vorgesehenen Mindestruhezeit. Entsprechend einer bereits erwähnten vorteilhaften Ausgestaltung ist die Manschette 4 während der gesamten Zeit, in welcher die Vorrichtung eingeschaltet ist, mit einem minimalen Überdruck beaufschlagt, um ihr Verrutschen zu verhindern. Erst mit der vollständigen Beendung der Überwachung im Schritt E erfolgt dann eine vollständige Entleerung der Manschette 4. In dem Gerät gespeicherte Daten können dann jedoch noch angezeigt, ausgelesen oder externen Geräten zur Weiterverarbeitung zur Verfügung gestellt werden. Mittels eines entsprechenden Bedienelementes, beispielsweise einer (nicht dargestellten) Reset-Taste, kann das Gerät in einen Grundzustand zurückgesetzt werden.

Der gesamte dargestellte Ablauf kann auch noch unter Einbeziehung von Verfahrensschritten zur Prüfung einer gegebenenfalls stattfindenden plötzlichen Beschleunigung des Probanden bzw. der Vorrichtung und einer in diesem Fall erfolgenden Unterbrechung der Messung erfolgen. Dann ist diese Prüfung auf Vorliegen einer Beschleunigung innerhalb des dargestellten Verfahrensregimes der eigentlichen Messung übergeordnet, jedoch dem Abbruchkriterium einer Zeitüberschreitung für das Aufpumpen der Manschette 4 untergeordnet.

### Liste der verwendeten Bezugszeichen

- 1: Wandlereinheit, Wandler bzw. Meßwandler
- 2: Wandlereinheit, Wandler (innere akustische Artefakte)
- 3: Wandlereinheit, Wandler (äußere akustische Artefakte)
- 4: Manschette, Druckmanschette
- 5: Kammer, Messkammer
- 6: Kammer, Ausgleichsvolumen
- 7, 7': Einheit zur Phasendrehung
- 8: Einheit zur Phasenverschiebung
- 9: Signaladdierer
- 10: Steuer- und Auswerteeinheit
- 11: Druckmesser
- 12: Anzeigeeinheiten
- 13: Einheit zur Speicherung, Speicher
- 14: Körperglied
- 15, 15; 15", 15''': Verstärker
- 16: Ventil
- 17: Pulsdetektor

## Patentansprüche

1. Vorrichtung zur nichtinvasiven Messung und/oder Überwachung des Blutdrucks, durch eine einmalige oder wiederholte, unter temporärer Speicherung der erhaltenen Werte erfolgende Ermittlung des systolischen und des diastolischen Blutdrucks eines Probanden, welche zumindest eine an einem Körperglied (14) anzulegende Manschette (4) mit einer pneumatisch befüllbaren, das Körperglied (14) zumindest teilweise umfassenden Kammer (5) zur wechselweisen partiellen Einengung und Entlastung eines Blutgefäßes, eine Pumpe zur pneumatischen Befüllung der Kammer (5), Mittel zur zeitlich gesteuerten Verringerung eines in der Kammer (5) infolge des Aufpumpens der Manschette (4) entstandenen Überdrucks, mehrere akusto-elektrische Wandler (1, 3), Einheiten zur Phasenverschiebung bzw. -drehung (7' 8) eines elektrischen Wechselsignals der Wandler (1, 3), einen Signaladdierer (9), einen Druckmesser (11) zur Messung des pneumatischen Drucks in der Kammer (5), Mittel zur Speicherung (13) und Anzeige von Messwerten (12), Mittel zur Bedienung der Vorrichtung, eine Steuer- und Auswerteeinheit (10), Mittel zur Spannungsversorgung der vorgenannten Einheiten sowie Mittel (2, 7) und/oder Mittel (6, 16) zur Eliminierung durch Bewegungen des Probanden hervorgerufener Störfaktoren umfasst und bei der die akusto-elektrischen Wandler (1, 3) zwei, jeweils immer mindestens einen Wandler umfassende Wandlergruppen ausbilden, von denen eine auf der dem Körperglied (14) abgewandeten Seite der dieses umfassenden Kammer (5) und die andere auf der dem Körperglied (14) zugewandten Seite der Kammer (5) angeordnet ist und wobei die elektrischen Signale der nicht am Blutgefäß anliegenden Wandlergruppe (3) nach einer durch die Einheit zur Phasendrehung (7') bewirkten Drehung ihrer Phase mit dem zuvor hinsichtlich seiner Phase durch die Einheit zur Phasenverschiebung (8) um die zur Phasendrehung benötigte Zeit verschobenen Signal der am Blutgefäß anliegenden Wandlergruppe (1) in dem Signaladdierer (9) zusammengeführt werden.

2. Vorrichtung nach Anspruch 1, **dadurch gekennzeichnet, dass** zur Eliminierung von bei der Bewegung des Probanden auftretenden inneren Artefakten eine mindestens einen elektro-akustischen Wandler (2) umfassende Wandlergruppe auf der dem Körperglied zugewandten Seite der Kammer (5), dem bei der Messung wechselweise partiell eingeengten Blutgefäß gegenüberliegend, angeordnet ist, deren aus der Wandlung detektierter akustischer Signale resultierende elektrische Signale zunächst einer Einheit zur Phasendrehung (7) zugeführt und nach erfolgter Phasendrehung gemeinsam mit den Ausgangssignalen der Einheit zur Phasendrehung (7') sowie der Einheit zur Phasenverschiebung (8) auf den Signaladdierer (9) geführt werden.

3. Vorrichtung nach Anspruch 1 oder 2, **dadurch gekennzeichnet, dass** im radial äußeren Bereich der Manschette (4) ein die Kammer (5) umgebendes und mit dieser über ein Ventil (16) verbundenes Ausgleichsvolumen (6) ausgebildet ist, wobei die Kammer (5), das Ausgleichsvolumen (6) und das Ventil (16) einen Regelungskreis zur Kompensation von durch das Anspannen und Erschlaffen der Muskulatur bei Bewegungen des Probanden in der Kammer (5) auftretenden Druckschwankungen bilden.

4. Vorrichtung nach Anspruch 3, **dadurch gekennzeichnet, dass** das die Kammer (5) zur wechselweisen partiellen Einengung und Entlastung des Blutgefäßes mit der Ausgleichskammer (6) unter Bildung eines Regelungskreises verbindende Ventil (16) als ein Ventil ohne Hilfsenergie ausgebildet ist.

5. Vorrichtung nach Anspruch 3, **dadurch gekennzeichnet, dass** das die Kammer (5) zur wechselweisen partiellen Einengung und Entlastung des Blutgefäßes (Messkammer) mit der Ausgleichskammer (6) verbindende Ventil (14) als ein von der Steuer- und Auswerteeinheit (10) steuerbares Ventil und der die Messkammer (5), das Ausgleichsvolumen (6), das Ventil (16) sowie Teile der Steuer- und Auswerteeinheit (10) umfassende Regelungskreis als PID-Regler ausgebildet ist.

6. Vorrichtung nach einem der Ansprüche 1 bis 5, **dadurch gekennzeichnet, dass** die Vorrichtung über Mittel für eine zeitzyklisch gesteuerte wiederholte automatisierte Ermittlung des systolischen und diastolischen Blutdrucks verfügt.

7. Vorrichtung nach einem der Ansprüche 1 bis 6, **dadurch gekennzeichnet, dass** die Vorrichtung über Mittel für eine hinsichtlich des Wiederholungsintervalls zufallsgesteuerte wiederholte automatisierte Ermittlung des systolischen und diastolischen Blutdrucks verfügt.

8. Vorrichtung nach einem der Ansprüche 1 bis 7, **dadurch gekennzeichnet, dass** die Vorrichtung über Mittel für eine automatisiert ereignisgesteuerte wiederholte Ermittlung des systolischen und diastolischen Blutdrucks verfügt, durch welche die zur Ermittlung der Blutdruckwerte erforderlichen Detektierungs- und Messvorgänge bei Feststellung einer vorgebbaren Erhöhung der Pulsfrequenz oder bei Feststellung einer Verringerung des Anstiegs der sich erhöhenden Pulsfrequenz gestartet werden.

9. Vorrichtung nach Anspruch 8, **dadurch gekennzeichnet, dass** die Vorrichtung einen zusätzlichen Pulsdetektor (17) zur Feststellung der Pulsfrequenz aufweist, der über die Steuer- und Auswerteeinheit (10) in der Weise mit den Mitteln zur Speicherung (13) von Messwerten gekoppelt ist, dass in der Steuer- und Auswerteeinheit (10) eine gegebenenfalls erfolgende Erhöhung der Pulsfrequenz, der jeweils im Verhältnis zur letzen Pulsfrequenzmessung sich ergebende Anstieg der Pulsfrequenz und ein innerhalb eines vorgebbaren zeitlichen Intervalls gegebener maximaler Anstieg der Pulsfrequenz abgeleitet werden.

10. Vorrichtung nach einem der Ansprüche 1 bis 9, **dadurch gekennzeichnet, dass** die Mittel zur Bedienung sowie zur Speicherung (13) und zur Anzeige (12) von Messwerten die Abfrage und Anzeige ausgewählter Messwerte zulassen.

11. Vorrichtung nach einem der Ansprüche 1 bis 10, **dadurch gekennzeichnet, dass** die Vorrichtung eine graphische Darstellung von Messwerten ermöglicht.

12. Vorrichtung nach einem der Ansprüche 1 bis 11, **dadurch gekennzeichnet, dass** die Vorrichtung Mittel zum Anschluss externer Geräte für das Auslesen und eine weitere Verarbeitung von Messwerten aufweist.

13. Vorrichtung nach einem der Ansprüche 1 bis 12, **dadurch gekennzeichnet, dass** die Vorrichtung über Mittel zum Anschluss externer Geräte verfügt, durch welche die Messung zur Ermittlung des systolischen und des diastolischen Blutdrucks gestartet und/oder die zeitliche Abfolge wiederholter Messungen gesteuert wird.

14. Vorrichtung nach einem der Ansprüche 1 bis 13, **dadurch gekennzeichnet, dass** die Vorrichtung über Mittel zur Unterstützung des Anlegens der Manschette an das Körperglied (14) in einer für die Detektion der Korottkoff-Geräusche günstigen Lage verfügt, wobei diese Lage durch eine akustische Detektion und Auswertung der durch das in einem Blutgefäß strömende Blut verursachten Geräusche vermittels der zur Anlage an das Blutgefäß bestimmten Wandlergruppe (1) akusto-elektrischer Wandler ermittelt und durch hierfür geeignete Signalisierungsmittel optischer oder akustischer Art kenntlich
